Europäisches Patentamt

European Patent Office

Office européen des brevets

(19)

(11) Publication number: 0 211 756

A1

## (12) EUROPEAN PATENT APPLICATION

(21) Application number: 86401702.5

(22) Date of filing: 30.07.86

(51) Int. Cl.⁴: C 07 K 15/14
G 01 N 33/569, C 12 Q 1/70
C 07 K 17/00, A 61 K 39/35

(30) Priority: 01.08.85 US 761246
01.08.85 US 761245
01.08.85 US 761248

(43) Date of publication of application:
25.02.87 Bulletin 87/9

(84) Designated Contracting States:
AT BE CH DE FR GB IT LI LU NL SE

(71) Applicant: MERCK & CO. INC.
126, East Lincoln Avenue P.O. Box 2000
Rahway New Jersey 07065(US)

(72) Inventor: Ellis, Ronald W.
1407 Edgevale Road
Overbrook Hills Pennsylvania 19151(US)

(72) Inventor: Neff, Beverly J.
401 Revere Drive
Harleysville Pennsylvania 19438(US)

(72) Inventor: Keller, Paul M.
2057 Spring Valley Road
Lansdale Pennsylvania 19446(US)

(72) Inventor: Emini, Emilio A.
Six Flaggs Manor Lane
Paoli Pennsylvania 19301(US)

(74) Representative: Ahner, Francis et al,
CABINET REGIMBEAU 26, avenue Kléber
F-75116 Paris(FR)

(54) Assay for varicella-zoster virus (VZV) antibodies.

(57) Antibodies in mammalian biological fluids to each of the three major VZV glycoprotein groups, gA, gB and gC, can be detected, for example, by isolating each viral glycoprotein from VZV-invected cells *in vitro*, adhering these to a plastic surface and contacting with a biological fluid containing the suspected antibodies. Subsequent treatment with a conjugate consisting of, for example, an antibody-enzyme complex (with specificity for either one, two or all three glycoprotein groups), followed by contact with an enzyme substrate and subsequent determination of the quantity of reacted enzyme substrate, i.e., the ELISA assay, provides a relative measure of the antibody(ies) present directed against VZV glycoproteins.

EP 0 211 756 A1

## TITLE OF THE INVENTION
ASSAY FOR VARICELLA-ZOSTER VIRUS (VZV) ANTIBODIES

## BACKGROUND OF THE INVENTION

Chickenpox is caused by varicella-zoster virus (VZV), a member of the herpes virus group, and occurs in persons with no prior or low level VZV immunity. The presence of VZV-specific antibodies can be demonstrated shortly after onset of disease, declines during convalescence, but remains detectable for many years and correlates with immunity to the disease. Chickenpox is highly contagious; over 90% of the population becomes exposed to VZV during the first two decades. The disease is highly morbid to the immunosuppressed and to those beyond the second decade. In most, if not all cases, VZV becomes latent in dorsal root ganglion cells. From this latent·state, VZV can reactivate and cause zoster

0211756

(shingles) even in the presence of specific antibodies, probably as a result of diminished cellular immunity. Management of contact between non-immune individuals and active varicella cases is necessary in some situations to prevent transmission to persons at increased risk of morbidity due to varicella.

VZV has been found to contain six major glycoproteins on its surface: gp105, gp92, gp83, gp62, gp57, gp55. These glycoproteins apparently are the products of three genes: gA (gp105), gB (gp62, gp57), and gC (gp92, gp83, gp55). The gC glycoproteins are the majority and most immunogenic of the VZV glycoproteins. Some monoclonal antibodies to gA and gB display complement-independent neutralization of viral infectivity in vitro, and monoclonal antibodies to gC display complement-dependent neutralization.

Presently known assays for the detection of antibodies to varicella-zoster virus (VZV) consist of methods for adhering virally infected cells or lysates of virally infected cells to a solid support, e.g., fluorescent antibody to membrane antigen, (FAMA), immune adherence hemagglutination (IAHA), enzyme-linked immunosorbent assay (ELISA), followed by contacting these with the biological fluid and then a second antibody conjugated to an enzyme or a fluorescent probe.

A commercially available varicella diagnostic device, VARICELISA[R] TEST KIT, by Whittaker M. A. Bioproducts of Walkersville, Maryland, utilizes an ELISA-type assay for IgG antibody to VZV in human serum.  The test utilizes total VZV-infected cell antigens.  The technical product bulletin is hereby incorporated by reference for its description of the device Catalog Number 30-3520 (plates) and in vitro procedure which can be adopted for use with the glycoprotein and methods of the subject invention.

The above referred-to tests detect a wide range of antibodies reactive with a variety of VZV antigens.  However, the tests are incapable of distinguishing among antibodies of groups reactive with the three families of VZV glycoproteins, gA, gB and gC, each of which is now known to stimulate the production of antibodies which can neutralize viral infectivity in vitro, or among antibodies reactive with VZV glycoproteins as opposed to other proteins.

## OBJECTS OF THE INVENTION

Accordingly, it is an object of the present invention to provide an improved assay capable of detecting subgroups of antibodies specific to purified gA, gB and gC VZV glycoproteins.  Another object is to provide a preparative method and diagnostic use for these purified glycoproteins and their controls as detecting antigens.  These and other objects of the present invention will be apparent from the following description.

## SUMMARY OF THE INVENTION

In accordance with this invention, there is provided a method for determining the presence of antibodies to varicella-zoster virus (VZV) gA, gB and gC glycoproteins in a host mammalian biological fluid comprising the steps of:

0211756

3492P/1142A — 4 — 17225Y

(a) permanently adhering at least one purified non-denatured, and serologically active gA, gB or gC VZV glycoprotein or mixture thereof, to a solid surface;

(b) permanently adhering purified, non-denatured and serologically active uninfected cell proteins or glycoproteins or mixture thereof which co-purify with VZV gA, gB or gC glycoproteins, to a solid surface

(c) contacting the adhered glycoprotein on said solid surface from step (a) and adhered protein or glycoprotein on said solid surface from step (b) with the biological fluid, wherein antibodies to the glycoprotein, if present in the fluid, bind to said glycoprotein forming a glycoprotein-antibody complex;

(d) contacting the adhered glycoprotein on said solid surface from steps (b) and (c) with an anti-immunoglobulin-indicator complex, directed against subclasses, total immunoglobulins or against specific immunoglobulin wherein said anti-immunoglobulin binds to said antibodies of said glycoprotein-antibody complex, if present from steps (b) or (c), thereby forming a glycoprotein-antibody-anti-immunoglobulin-indicator complex, wherein said indicator provides a measurable response of the binding event; and

(e) measuring the indicator response after conducting step (d) and correcting the response to the individual VZV glycoprotein or mixture thereof by subtraction of the response to its respective uninfected cell protein or glycoprotein control.

Particularly provided is a method for determining in vitro the presence of human antibodies to varicella-zoster virus (VZV) gA, gB, and gC glycoproteins in human serological fluid comprising the steps of:

(a) permanently adhering gA, gB or gC purified, non-denatured and serologically active VZV glycoproteins derived from VZV-infected cells to a solid polystyrene surface;

(b) permanently adhering purified, non-denatured and serologically active uninfected cell proteins or glycoproteins or mixture thereof which co-purify with VZV gA, gB or gC glycoproteins, to a solid polystyrene surface;

(c) contacting the resulting adhered glycoprotein on said surface from steps (a) and (b) with the serological fluid, wherein antibodies to the glycoprotein, if present in the fluid, bind to said glycoprotein forming a glycoprotein-antibody complex;

(d) contacting the adhered glycoprotein on said surface from step (c) with an anti-human-immunoglobulin, derived in response to said antibodies, and conjugated with alkaline phosphatase, wherein said anti-immunoglobulin binds to said antibodies of said glycoprotein-antibody complex, if present;

(da) contacting the complex from step (c) with p-nitrophenyl-phosphate solution; and

(e) measuring the absorbance due to p-nitrophenol in alkaline solution after development of the enzyme substrate solution in step (da) and measuring the absorbance after development of the enzyme substrate solution and correcting the response to the individual VZV glycoprotein or mixture thereof by subtraction of the response to its respective uninfected cell protein or glycoprotein control.

Also disclosed are the following glycoproteins:

VZV gA glycoprotein in its purified, non-denatured, serologically active form;

VZV gB glycoprotein in its purified, non-denatured, serologically active form;

VZV gC glycoprotein in its purified, non-denatured, serologically active form;

purified infected cell VZV glycoprotein purified by lectin-affinity chromatography, and containing gA and/or gB and/or gC glycoproteins.

uninfected cell proteins in purified, non-denatured, serologically active form which co-purify with VZV gA, gB or gC from VZV-infected cell lysates by immune-affinity chromatography;

purified uninfected cell glycoproteins which co-purify with VZV glycoproteins from VZV-infected cell lysates by lectin affinity chromatography.

Further disclosed are the following VZV diagnostic devices:

a diagnostic device comprising VZV gA glycoprotein in its purified, non-denatured, serologically active form permanently adhered to a solid surface;

a diagnostic device comprising VZV gB glycoprotein in its purified, non-denatured, serologically active form, permanently adhered to a solid surface;

a diagnostic device comprising VZV gC glycoprotein in its purified, non-denatured, serologically active form, permanently adhered to a solid surface;

a diagnostic device comprising VZV gA or gB or gC glycoproteins, or mixture thereof, in their purified, non-denatured, serologically active form, at least two of said glycoproteins being present, and permanently adhered to a solid surface; and

a diagnostic device comprising uninfected cell proteins in purified, non-denatured, serologically active form which co-purify with VZV ga, gB or gC from VZV-infected cell lysates by immune-affinity chromatography, and permanently adhered to a solid surface;

a diagnostic device comprising purified infected cell VZV glycoprotein, purified by lectin-affinity chromatography, and containing gA, gB and gC glycoproteins, in their non-denatured, serologically active form, permanently adhered to a solid surface;

a diagnostic device comprising purified uninfected cell glycoproteins purified by lectin-affinity chromatography and containing glycoproteins in their non-denatured, serologically active form, permanently adhered to a solid surface.

## BRIEF DESCRIPTION OF THE DRAWINGS

Fig. 1 illustrates the electrophoretic analysis of immunoprecipitations of gA, gB and gC VZV polypeptides with A1, B1 and C1 monoclonal antibodies.

Fig. 2 illustrates Western blot analysis of the reactivity of C5 monoclonal antibody with VZV polypeptides.

Fig. 3 illustrates a fluorograph of SDS-polyacrylamide gels showing antigenic distinctiveness among gA, gB and gC VZV glycoproteins as demonstrated by cross-clearing immunoprecipitation.

## DETAILED DESCRIPTION OF THE INVENTION AND PREFERRED EMBODIMENTS

The present invention relates to an assay for detecting mammalian antibodies to specific gA, gB or gC VZV glycoproteins in biological fluid and, more particularly, to a highly sensitive enzyme-linked assay (ELISA) for detecting these antibodies in biological fluid, especially human biological fluid. According to the present invention, VZV gA, gB, gC glycoproteins, or mixture thereof, from a solution of known protein concentration, are permanently adhered to a solid surface capable of adsorbing the glycoprotein, e.g., a plastic surface. The nomenclature of the glycoproteins in the art is now assigned as: gpI (gpC), gpII (gpB) and gpIII (gpA) according to the article: J. of Virology, March 1986, pp. 1195-1197 by Davison et al. hereby incorporated by reference. However the gpA, gpB and gpC nomenclature is used herein. By the term "permanently adhered", as used herein, is meant that the glycoprotein cannot be easily removed from the solid surface in a non-denaturing manner, such as by simply washing with water or an aqueous buffered solution as in chromatographic elution.  More drastic conditions are needed to remove the glycoprotein, such as boiling in water or an organic solution, all of which denature the glycoprotein in the removal process.

By the term "non-denatured" as used herein is meant reactive in solution with antibodies which are capable of binding to the native non-purified glycoprotein.

By the term "serologically active", as used herein is meant capable of eliciting, upon injection to an animal, antibodies capable of binding to the native non-purified glycoprotein.

By the term "fluid", as used herein is meant a fluid, e.g., blood, serum or plasma, derived from a living organism.

The glycoproteins and controls which can be utilized as test antigens are as follows:

1.  <u>VZV gA glycoproteins ("gA")</u>, purified from VZV-infected human cells (e.g., MRC-5 cells infected with KMcC strain live attenuated VZV virus 41071, as described in Proc. Soc. Exp. Biol. Med., <u>166</u>: 339-347) by immune-affinity chromatography utilizing monoclonal antibody Al reactive with VZV gA glycoproteins.  The MRC-5 human lung fibroblast cells are publicly available, ATCC-171; also WI-38 human lung fibroblast cells, ATCC-75, as well as other non-tumorigenic human lung fibroblast cells can be utilized with equivalent results.  The KMcC strain can be substituted by any VZV strain, wild type or attenuated, e.g. varicella zoster Oka strain, ATCC VR795, publically available, with equivalent results as well as the SmithKline varicella vaccine, publicly available in Europe, which is derived from the Oka wild type strain.  The preparation of monoclonal antibody A1 is given in Example 1 and of VZV gA glycoprotein in Example 3.  The identity of this glycoprotein in denatured form is further described by the following references which are hereby incorporated by reference for this purpose: Keller et al., J. Virology 52: 293 (1984) i.e., gp105; Grose et al., Infect. Immun. 40: 381 (1983) i.e., gp118, who isolated gA in its purified form; Shiraki et al., J. Gen. Virol. 61: 255 (1982) i.e., gp1; and Forghani et al., J. Virol. 52: 55 (1984) i.e., gp118.

2.  Uninfected cell polypeptides ("gA control") obtained from uninfected, e.g., MRC-5 cell line, are purified by immune-affinity chromatography utilizing the monoclonal antibody A1 described above reactive with the VZV gA glycoproteins substantially by the method described in Eisenberg et al., J. Virol. 41: 1099 (1982), as in Example 5.

3.  VZV gB glycoproteins ("gB") are purified from VZV-infected cells by immune-affinity chromatography utilizing a monoclonal

antibody B1 reactive with the VZV gB glycoproteins.  The preparation of monoclonal
antibody B1 is given in Example 1 and of gB
VZV glycoprotein in Example 2.  The identity
of these glycoproteins in denatured form is
further described by the following references
which are hereby incorporated by reference
for this purpose:  Keller et al., ibid i.e.,
gp115, gp62, gp57; Okuno et al., Virology
129: 357 (1983) i.e., gp2, gp5; Grose et
al., gp140, gp66, "disulfide linked dimer";
and Forghani et al., ibid, 120K, 118K,
64-65K.

4.  Uninfected cell polypeptides ("gB control")
are purified by immune-affinity chromato-
graphy utilizing monoclonal antibody B1
described above reactive with the VZV gB
glycoproteins,substantially by the method
described in Example 6.

5.  VZV gC glycoproteins ("gC") are purified
from VZV-infected cells by immune-affinity
chromatography utilizing monoclonal antibody
C1 reactive with the VZV gC glycoproteins.
The preparation of monoclonal antibody C1 is
given in Example 1 and of gC VZV glycoprotein
in Example 4.  The identity of these glycoproteins in denatured form is further
described by Keller et al., ibid i.e., gp92,
gp83, gp52, gp45; Grose et al., Infect.
Immun. 40: 381 (1983) i.e., gp98, gp63;
Okuno et al., ibid i.e., gp3, gp4, gp6; and
Forghani et al., ibid, 90K, 80K, 60K.

6.  Uninfected cell polypeptides ("gC control") are purified by immune-affinity chromatography utilizing monoclonal antibody Cl described above reactive with the VZV gC glycoproteins substantially by the method described in Example 7.

7.  VZV total glycoproteins ("lectin") are purified from VZV-infected cells by lectin-affinity chromatography utilizing an immobilized lectin type which is broadly cross-reactive with all VZV glycoproteins. The method is described in Hayman et al., FEBS Lett., Vol. 29, 185 (1973), as in Example 8.

8.  Uninfected cell total glycoproteins ("lectin control") are purified from uninfected cells by lectin-affinity chromatography utilizing the same immobilized lectin and procedure as described in 7 above, as in Example 9.

It is the presence of the above-described glycoproteins on the surface of the VZV virion which stimulates immunity to the virus. This immunity can be in the form of humoral immunity, i.e., the formation of antibodies, or cellular immunity. The gA, gB and gC antigens described above, which are distinguishable on the basis of physical and chemical characteristics and reactivity with different monoclonal antibodies, can be used as diagnostic reagents in assays to measure cell-mediated immunity by the herein-described method, diagnostic devices and purified VZV glycoproteins. Examples of such assays include delayed-type hypersensitivity (skin test antigen) and _in vitro_ T-cell proliferation assays.  Moreover, the purified gA, gB or gC VZV glycoproteins, either singly or in combinations, can also be purified for use and utilized as diagnostic reagents in alternative procedures such as immune-affinity chromatography utilizing polyclonal monospecific antibodies or polyclonal human acute- or convalescent-phase sera.

The subject method described herein can be generally carried out by permanently adhering the glycoproteins described above in their purified, non-denatured and serologically active form to a solid surface, being a strip or typically a well of a polystyrene multi-well assay plate, by incubation at about 4° to 8°C, typically for about 18 to 48 hours, to coat the surface with glycoproteins.  The surface is subsequently drained, washed with a suitable solution, e.g., phospate-buffered saline (PBS; 0.15M NaCl, 0.01M $Na_2HPO_4$, pH 7.2) with 0.05% Tween 20[R].  Serial dilutions of the biological fluid to be tested for the presence of antibodies to VZV glycoproteins are made in PBS with 0.05% Tween 20[R] containing 1% fetal bovine serum and "globulin-poor" goat serum which is prepared from normal goat serum, Hazleton Labs, Denver, Pennsylvania as follows:

a.   dilute normal goat serum 1:2 with $H_2O$;

b.   mix with an equal volume of saturated $(NH_4)_2SO_4$ which is added dropwise with constant stirring;

c.   store overnight at 4°C;

d.   centrifuge 10° at 1500 rpm;

e. discard precipitate;

f. supernatant is filter sterilized.

These serial dilutions of virological fluid are contacted to the washed surface and incubated for from about 0.5 hours to 2.0 hours at from about 25°C to about 40°C. The wells are then washed thoroughly with PBS with 0.05% Tween 20$^R$ to remove unbound antibodies if present. The wells then are contacted with typically an anti-immunoglobulin-enzyme conjugate and allowed to incubate at 37°C for 1 hour. The unbound conjugate is washed off with the above PBS/Tween solution. Next typically is added an enzyme substrate solution, under the same conditions as the previous step, except that the substrate incubation is performed at 18°C to 28°C for about one hour and then stopped by the addition of base (1N NaOH). The optical density (A) of the wells is then measured at 405-490 nm. The readings then are corrected for their respective controls obtained by conducting the identical method on non-infected MRC-5 cell glycoproteins; e.g., antibodies (gA)= absorbance (gA) [A(gA)] - A (gA control); antibodies (gB) = A (gB) - A (gB control); antibodies (gC) = A (gC) - A (gC control); antibodies (total glycoproteins) = A (lectin) - A (lectin control).

In the method, the mammalian biological fluid can be murine, bovine, caprine, human or any mammal which is active towards varicella-zoster virus. Preferably, the mammalian biological fluid used in the method is human.

The fluid itself to be tested can be whole blood, plasma or serum of the mammal. Preferably, the serum is utilized.

The solid surface used in the subject method can be of any synthetic polymer including polystyrene, nylon, polyester, polyvinylchloride, polyacrylonitrile, and the like or other chemically non-reactive solid particle composed of substances such as glass or carbon. Preferred is polystyrene and particularly preferred is smooth polystyrene as in a commercially available multi-well assay plate as for example, commercially made by Nunc, Linbro or Dynatech.

The glycoprotein-antibody complex formed is a result of the specific interaction between serologically active antibodies and their respective gA, gB, gC, or mixture thereof, antigen glycoproteins; i.e., gA antibody reacts with gA antigen glycoprotein.

The anti-immunoglobulin-indicator complex contains an anti-immunoglobulin which is derived from a different host than the mammalian biological fluid host source. When testing for human VZV antibodies, the anti-immunoglobulin is derived from a different source, e.g., mouse or rabbit, into which the human antibodies have been introduced to produce the anti- immunoglobulin which can be isolated and purified by known techniques in the art. A preferred anti- immunoglobulin is goat or rabbit anti-human IgG. Anti-immunoglobulins can also be directed against IgM or IgA for the purpose of subclass antibody determinations. Other indicator complexes can include conjugates of staph A protein or other substances which specifically bind antibody.

The indicator of the complex can be a phosphorescent probe or agent; fluorescent agent such as rhodamine or fluorescine; radiolabel such as $I^{125}$, or enzyme-linked immunosorbent assay (ELISA) reagents such as alkaline phosphatase, biotin-avidin system, horseradish peroxidase, or beta-D-galactose. Preferably, an ELISA reagent is utilized because of the high sensitivity and specificity.

A preferred enzyme reagent in the method is alkaline phosphatase.

The enzyme substrate which can be used is any substrate which is operated upon by the enzyme to produce a measurable response which is indicative of the binding events between the antibody and glycoprotein, and between the antibody-glycoprotein complex and the anti-immunoglobulin-enzyme or other indicator complex. The response is generally a change in the optical density (A) or absorbance of the enzyme substrate solution, generally being a color change in the 400-800 mμ visible region. The response should be spectrophotometrically measured with a standard visible or ultraviolet spectrophotometer.

Enzyme substrates which can be used include 3-amino-9-ethylcarbazole, 3,3',5,5'-tetramethylbenzidine (TMB), o-phenylenediamine (OPD), 2,2'-azinodi[3-ethylbenzthiazoline-sulfonate] (ADTS), and p-nitrophenylphosphate. Preferred enzyme substrate for use in the method is p-nitrophenylphosphate.

The response if a radiolabel can be quantitated by suitable radiation counters or scintillators for α,ß or gamma radiation.

If a phosphorescent or fluorescent probe, then suitable spectrophotometers known in the art can be employed.

3492P/1142A                    - 17 -                    17225Y

In the preferred method herein, the enzyme alkaline phosphatase operates on p-nitrophenyl phosphate to release p-nitrophenol which in alkaline solution absorbs at 405 nm and gives an indirect measure of the extent of the binding event for visual read-out.

In the method, thorough washing of the glycoprotein-antibody complex is required to remove all unbound antibodies prior to complexing with the anti-immunoglobulin-indicator or other indicator complex to avoid misleading high readings.

Similarly, thorough washing is required of the glycoprotein-antibody-anti-immunoglobulin-enzyme complex or glycoprotein antibody indicator to remove unbound anti-Ig-enzyme complex prior to contacting with the enzyme substrate.

As described above, the gA, gB and gC controls are obtained by carrying out the ELISA procedure using the glycoprotein from non-VZV infected cell lysates of, e.g., the MRC-5 cell lines. The procedure for preparing the glycoprotein from non-VZV-infected cell lysates is described above.

In the method, the glycoprotein used can be individually gA, gB or gC, or a mixture of any two or all three, in varying proportions. Preferably, the glycoprotein is used individually, e.g., gA.

The total VZV-infected cell glycoprotein, purified by lectin chromatography can also be used as the permanently adhered glycoprotein in the assay.

VZV, gA, gB and gC glycoproteins in their purified, non-denatured, serologically active form also are disclosed as novel reagents and compositions.

Uninfected cell proteins which co-purify with VZV gA, gB and gC from VZV-infected cell lysates by immune affinity chromatography in their purified, nondenatured, serologically active form also are disclosed as novel reagents and compositions.

The purification and isolation of these materials by immune-affinity chromatography is given in the examples.

Also disclosed is total VZV infected cell glycoprotein, purified by lectin-affinity chromatography and containing gA, gB and gC glycoproteins. This purified mixture can also be utilized in the subject method.

Also disclosed is total uninfected cell glycoprotein, purified by lectin-affinity chromatography. This purified mixture also can be utilized in the subject method.

The purification and isolation of these materials by lectin-affinity chromatography is given in the examples.

Also disclosed are diagnostic devices comprising purified, non-denatured, serologically active VZV gA, gB or gC glycoprotein or mixtures thereof, permanently adhered onto a solid, e.g., synthetic polymer, surface, as described herein, preferably being polystyrene.

Also disclosed are diagnostic devices comprising purified, non-denatured, serologically active uninfected cell proteins which co-purify with VZV gA, gB or gC from VZV-infected cell lysates by immune-affinity chromatography removed by being permanently adhered onto a solid, e.g., synthetic polymer surface, as described herein, preferably being polystyrene.

Total cell VZV infected glycoprotein, purified by lectin affinity chromatography, is also a useful device, permanently adhered to a solid surface.

Total uninfected cell glycoprotein purified by lectin-affinity chromatography is useful, permanently adhered to a solid surface.

The following examples illustrate the present invention without, however, limiting the same thereto:

REAGENTS USED IN THE EXAMPLES:

1. Goat anti-human immunoglobulin coupled to alkaline phosphatase

2. Mouse anti-human immunoglobulin coupled to alkaline phosphatase both derived from Tago, Inc., Burlingame, California 94010

3. Caprine serum albumin is better referred to as "globulin-poor" goat serum which is prepared from normal goat serum, commercially available from Hazleton Labs, Denver, Pennsylvania and formulated as follows:

   a.  dilute normal goat serum 1:2 with $H_2O$

   b.  mix with an equal volume of saturated $(NH_4)_2SO_4$ which is added dropwise with constant stirring

   c.  store overnight at 4°C

   d.  centrifuge 10° at 1500 rpm

   e.  discard precipitate

   f.  supernatant is filter sterilized.

## EXAMPLE 1

The following procedure for preparing monoclonal antibodies to the gA, gB and gC glycoproteins is taken from Keller et al., ibid., which is hereby incorporated by reference for this particular purpose.

Ten-week-old BALB/c mice were immunized subcutaneously at multiple sites with 20 µg (measured as described in the reference: Lowry, O. H., N. J. Rosebrough, A. L. Farr, and R. J. Randall, 1951, "Protein measurement with the Folin phenol reagent", J. Biol. Chem. 193:265-275) of purified virus (VZV strain KMcC described in: Neff, B. J., R. E. Weibel, V. M. Villarejos, E. B. Buynak, A. A. McLean, D.H. Morton, B. S. Wolanski, and M. R. Hilleman, 1981, "Clinical and laboratory studies of KMcC strain live attenuated varicella virus (41071)", Proc. Soc. Exp. Biol. Med. 166:339-347) emulsified in complete Freund's adjuvant, followed 2 weeks later by intraperitoneal boosting with 25 µg of purified VZV without adjuvant. All sera assayed 2 weeks after the second injection had antivirus titers of >400 and anticell titers of <50 in the live cell membrane fluorescence (FAMA) assay and reacted with VZV proteins in Western blots of infected cell extracts (data not shown). One month after the second immunization mice were injected intravenously with 25 µg of purified VZV without adjuvant. Three days later, spleens were removed from the two mice with the highest anti-VZV titers. Spleen cells were fused with SP2/0 mouse myeloma cells (Institute for Medical Research), and hybridoma cells were plated and cultured by previously published procedures:  Oi, V. T., and L. A. Herzenberg, 1980, "Immunoglobulin-producing hybrid cell lines", p. 351-372, in B. B. Mishell and S. M. Shiigi (ed.).  Selected methods in cellular immunology W. H. Freeman & Co., Inc., San Francisco.  Two weeks later, supernatant fluids from wells of actively growing cells were tested for anti-VZV reactivity by the FAMA assay.  Cells secreting antibodies reactive with VZV membrane antigens were cloned by limiting dilution and expanded into ascites fluids.  In general, two monoclones from each polyclone were used to produce ascites fluids.  No differences in the specificities of any two "sister" clones were observed.

Ten independent monoclonal antibodies in ascites fluids were produced which recognized VZV membrane antigens.  When tested for reactivity to VZV-infected MRC-5 cells, FAMA titers ranged from 1:32,000 to 1:256,000, whereas none of the antibodies reacted by FAMA to uninfected cells (Table 1). Antibody subclasses were characterized (Table 1). The antibody concentration in each ascites fluid was ca. 2 mg/ml.

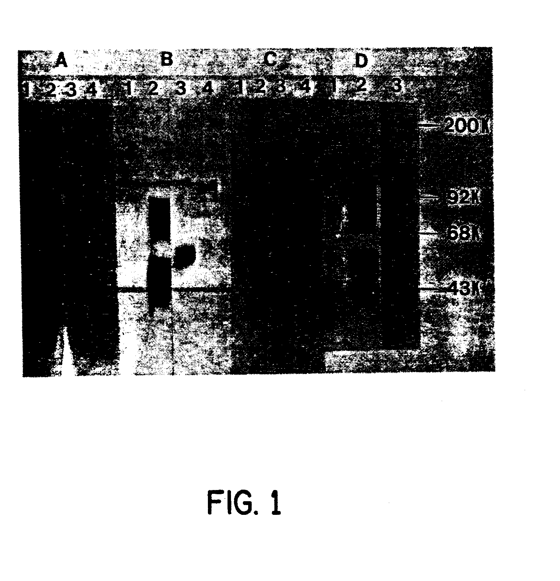

The serological specificities of the monoclonal antibodies were analyzed by immunoprecipitation.  Antibodies, as well as control ascites fluid, were reacted with [$^{35}$S]methionine-labeled infected cell extracts, [$^{14}$C]glucosamine-labeled infected cell extracts, and [$^{35}$S] methionine-labeled purified virions.  Sodium dodecyl sulfate (SDS)-polyacrylamide gel electrophoresis profiles are presented (Fig. 1A to C) and the interpretations of these profiles are summarized (Table 1). We observed that monoclonal antibodies C1 to C8 displayed virtually identical reactivities to VZV polypeptides (with the exceptions noted in footnote f of Table 1); thus, the profile displayed with antibody C8 (Fig. 1A2, B2, and C2) is representative of the C1 to C8 group. Three patterns of reactivity among the 10 monoclonal antibodies were observed. (i) Clone A1 antibodies recognized a single polypeptide termed gp105 (105,000-molecular-weight glycoprotein) from both infected cells and virions. (ii) Clone B1 antibodies recognized p110 (nonglycosylated), gp115, gp62, and gp57 from infected cells and only gp62 and gp57 from the virion. (iii) The eight group C antibodies recognized gp45 from infected cells only and gp92, gp83, and gp52 from both infected cells and virion.

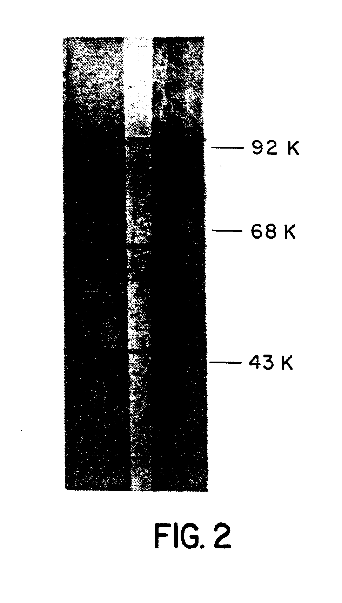

To characterize further the specificities of the monoclonal antibodies, both purified virus and infected cell extracts were denatured and resolved on Western blots, and ascites fluids were tested for reactivity. Neither A1 nor B1 reacted with any polypeptide, whereas seven of the eight group C antibodies reacted with the entire group of cellular antigens, gp92, gp83, gp52, and gp45. From purified virions, gp92 and gp83 predominated in reactivity, as was the case in immunoprecipitation analyses (Fig. 1C2). Representative profiles with one of the seven reactive group C antibodies are shown (Fig. 2). Significantly, the Western blot data reinforce the grouping of various polypeptides, i.e., gp92, gp83, gp52, and gp45 to a single family of antigens by ruling out possible nonspecific coprecipitations. It is noteworthy that all seven group C antibodies . reactive in Western blots also were positive by immunoprecipitation. This is in contrast to an earlier study, in which Western blot screening alone actually detected a greater number of monoclonal antibodies than did immunoprecipitation screening alone: Braun, D. K., L. Pereira, B. Norrild, and B. Roizman, 1983, "Application of denatured, electrophoretically separated, and immobilized lysates of herpes simplex virus-infected cells for detection of monoclonal antibodies and for studies of the properties of viral proteins", J. Virol. 46:103-112.

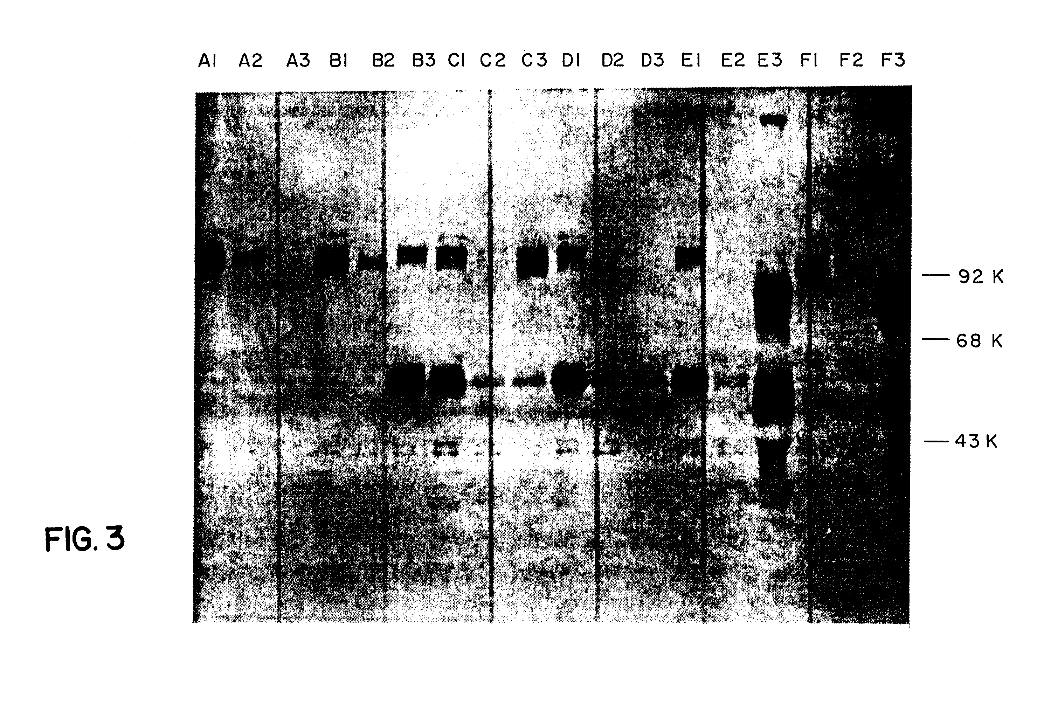

Several of the polypeptides defined by immunoprecipitation analysis exhibited similar electrophoretic mobilities. To investigate further the antigenic distinctions and possible serological groupings among viral gene products, cross-clearing immunoprecipitations were performed among the monoclonal antibodies with [$^{35}$S]methionine-labeled infected cell extracts (Fig. 3). After two clearings of the extract with A1, antibody A1 did not immunoprecipitate any additional gp105 (Fig. 3A3), whereas antibody B1 immunoprecipitated gp115, p110, and gp62 efficiently from the A1-cleared extract (Fig. 3B3). The converse experiment, with antibody B1 followed by A1 (lane groups C and D), as well as with antibody C6 (lane groups E and F), demonstrates similar distinctions. Therefore, our monoclonal antibodies define three families of antigenically distinct glycoproteins, which we term gA, gB and gC.

A cursory comparison between the glycoprotein species resolved above and those reported
elsewhere (Grose, C., 1980, "The synthesis of
glycoproteins in human melanoma cells infected with
varicella-zoster virus", Virology 101:1-9; Grose, C.,
1983, "Zoster in children with cancer:  radioimmune
precipitation profiles of sera before and after
illness", J. Infect. Dis. 147:47-56; Shemer, Y., S.
Leventon-Kriss, and I. Sarov, 1980, "Isolation and
polypeptide characterization of varicella-zoster
virus", Virology 106:133-140; Shiraki, K., T. Okuno,
K. Yamanishi, and M. Takahashi, 1982, "Polypeptides
of varicella-zoster virus (VZV) and immunological
relationship of VZV and herpes simplex virus (HSV);
J. Gen Virol. 61:255-269; Zweerink, H., and B. J.
Neff, 1981, "Immune response after exposure to
varicella zoster virus:  Characterization of virus-
specific antibodies and their corresponding antigens",
Infect. Immun. 31:436-444.) suggested that our mono-
clonal antibodies in toto had detected all major VZV
glycoproteins.  To address this question directly,
nonimmunoprecipitated [$^{14}$C]glucosamine-labeled
virions were lysed and electrophoresed (Fig. 1D1).
Also, [$^{14}$C]glucosamine-labeled infected cell
extracts were immunoprecipitated with a high-titer
human convalescent zoster serum (Fig. 1D2).  The
major detectable glycoprotein species in these two
experiments approximately coincided with the sum of
the gA, gB, and gC bands detected by the monoclonal
antibodies.  The existence of other minor glycoprotein
bands is not ruled out by this analysis.  Nevertheless, our analysis with monoclonal antibodies strongly suggests that gA, gB, and gC are the three major VZV glycoprotein genes.

Monoclonal antibodies were tested for biological activity against VZV by the neutralization assay (Table 1). Clone A1 antibodies neutralized strongly in the absence of complement. The eight monoclonal antibodies reactive with gC polypeptides neutralized only in the presence of complement. Our gB-reactive antibody failed to neutralize. However, we obtained a monoclonal antibody from C. Edson (Tufts University Boston, MA) for which we could demonstrate serological reactibility in immuno-precipitations identical to clone B1 antibodies (data not shown: reactive with gp63, gp125 in the nomenclature of Edson et al. [C. Edson, submitted for publication]). These antibodies neutralize infectivity in the absence of complement (data not shown; Edson, submitted for publication). Therefore, all three major VZV glycoprotein genes encode polypeptides with neutralization epitopes. This result is not unlike that found in the herpes simplex virus system, in which four distinct glycoprotein genes encode glycopeptides with neutralization epitopes (Balachandran, N., D. Harnish, R. A. Killington, S. Bacchetti, and W. E. Rawls, 1981, "Monoclonal antibodies to two glycoproteins of herpes simplex virus type 2", J. Virol. 39:438-446; Pereira, L., D. Dondero, B. Norrild, and B. Roizman, 1981, "Differential immunologic reactivity and processing of glycoproteins gA and gB of herpes simplex virus types 1 and 2 made in Vero and HEp-2 cells", Proc. Natl. Acad. Sci. U.S.A. 78:5202-5206; Pereira, L., T.

Klassen, and J. R. Baringer, 1980, "Type-common and type-specific monoclonal antibody to herpes simplex virus type 1", Infect. Immun. 29:724-737; Showalter, S.D., M. Zweig, and B. Hampar, 1981, "Monoclonal antibodies to herpes simplex virus type 1 proteins, including the immediate-early protein ICP 4", Infect. Immun. 34:684-692).

The differential representations of various glycoproteins among labeled cell extracts and virions enable us to suggest certain hypotheses about the relationships of the different polypeptide species. (i) The gA gene product gp105 probably corresponds to gp115 (gp1) of Shiraki et al. (ibid) and to gp118 of Grose et al. (Grose, C., D. P. Edwards, W. E. Friedrichs, K. A. Weigle, and W. L. Mcguire, 1983, "Monoclonal antibodies against three major glycoproteins of varicella-zoster virus", Infect. Immun. 40:381-388) who report that a monoclonal antibody to gp118 also neutralizes in the absence of complement. (ii) The gB gene products gp62 and gp57 probably represent the final processed forms of gB in the virion, since gp115 and p110 are present only in infected cells. Furthermore, the nonglycosylated p110 might represent a precursor to gp115. Similar conclusions have been reached by others (Edson, submitted for publication; gp115, gp106, and gp64 in reference: Okuno, T., K. Yamanishi, K. Shiraki, and M. Takahashi, 1983, "Synthesis and processing of glycoproteins of varicella-zoster virus (VZV) as studied with monoclonal antibodies to VZV antigens", Virology 129:357-368, although each group detected only a single virion glycoprotein species of molecular weight 63,000 to 64,000 and one group detected two higher-molecular-weight glycoproteins (Okuno et al, ibid.). Similar pulse-chase relationships have been found in herpes simplex virus type 2 (Balachandran, N., et al. supra.; Pereira, L. supra). (iii) The gC gene products gp92, gp83, gp52, and gp45 represent the predominant viral glycoprotein species based on SDS-polyacrylamide gel electrophoresis of purified [$^{14}$C]glucosamine-labeled virions as well as on immunoprecipitations with convalescent zoster sera. They appear to be the most immunogenic glycoproteins based upon the ease of isolation of monoclonal antibodies to gC, 8 of 10 in this report, 4 of 5 in another report (gp98, gp63, gp55, and gp45 [Grose, et al., Infect. Immun., supra], and 8 of 12 in another report (gp94, gp83, gp55, and gp45 [Okuno, T. et al., supra]). The gp92 and gp83 species seem to represent the mature virion polypeptides, as deduced by their overrepresentation relative to gp55 in virions. It is noteworthy that monoclonal antibodies to gC detect more than one glycoprotein, an observation that has been made in other members of the herpes virus family, e.g., herpes simplex virus (Balachandran, N., supra; Eberle, R., and R. J. Courtney, 1982, "Multimeric forms of herpes simplex virus type 2 glycoproteins", J. Virol. 41:438-351; Pereira, L. et al., Infect. Immun., supra), Epstein-Barr virus (Edson, C. M., and D. A. Thorley-Lawson, 1983, "Synthesis and processing of the three major envelope glycoproteins of Epstein-Barr virus", J. Virol. 46:547-556; Strnad, B. C., T. Schuster, R. Klein, R. F. Hopkins III, T. Witmer, R.

H. Neubauer, and H. Rabin, 1982, "Production and characterization of monoclonal antibodies against the Epstein-Barr virus membrane antigen", J. Virol. 41:258-264:) and cytomegalovirus (Pereira, L., M. Hoffman, D. Gallo, and N. Cremer, 1982, "Monoclonal antibodies to human cytomegalovirus: three surface membrane proteins with unique immunological and electrophoretic properties specify cross-reactive determinants", Infect. Immun. 36:924-932.). Since these cross-reactions do not appear to be nonspecific in nature, the multiple polypeptide forms could be processes from related but distinct precursors, could share pulse-chase relationships, or could represent heterogeneities in glycosylation patterns.

These monoclonal antibodies to gA, gB and gC can also be used to quantitate VZV glycoproteins by binding the viral antigens to an appropriate substrate, e.g. nitrocellulose, incubating the bound antigen with the monoclonal antibody, and subsequently treating this complex with an anti-antibody/enzyme complex, followed by contact with an enzyme substrate and subsequent determination of the quantity of reacted enzyme substrate.

TABLE 1
Specificity of the Reactivities of Monoclonal Antibodies

| Clone[a] | Antibody[b] isotype | Immunoprecipitation[c] | | Western blot[c] | | Antigen group recognized | Neutralization titer[d] | | FAMA titer[e] |
| | | Infected cell extracts | Purified virions | Infected cell extracts | Purified virions | | C' present | C'absent | |
| A1 | IgG2a | gp105 | gp105 | None | None | gA | 1:32,000 | 1:32,000 | 1:32,000 |
| B1 | IgG1 | p110, gp115, gp62, gp57 | gp62, gp57 | None | None | gB | 1:100 | 1:100 | 1:64,000 |
| C1 | IgG1 | gp92, gp83, gp52, gp45 | gp92, gp83, gp52 | gp92, gp83, gp52, gp45 | gp92, gp83 gp52 | gC | 1:4,000 | 1:100 | 1:256,000 |
| C2 | IgG1 | | | | | | 1:8,000 | 1:100 | 1:64,000 |
| C3[f] | IgG2a | | | | | | 1:16,000 | 1:100 | 1:256,000 |
| C4 | IgG1 | | | | | | 1:8,000 | 1:100 | 1:128,000 |
| C5[f] | IgG1 | | | | | | 1:4,000 | 1:100 | 1:32,000 |
| C6 | IgG2a | | | | | | 1:4,000 | 1:100 | 1:64,000 |
| C7[c] | IgG2b | | | | | | 1:8,000 | 1:100 | 1:254,000 |
| C8 | IgG1 | | | | | | 1:16,000 | 1:100 | 1:64,000 |

Footnotes a-g

## Footnotes

a   Hybridoma cells were cloned by limiting dilution onto irradiated (10,000 rads from a $^{60}$Co source) MRC-5 cells in 96-well plates.  For production of antibody in ascites fluid, mice primed with pristane (2, 6, 10, 14,-tetramethyl-pentadecane) were injected with 4 x $10^6$ cells per mouse.  Approximately 10 days later, ascites fluid was collected.

b   Immunoglobulin subclass was determined by immunodiffusion assay against goat monospecific antisera to mouse immunoglobulin G1 (IgG1), IgG2a, IgG2b, and IgG3 (Research Products International Corp.).

c   Antibodies in ascites fluids were tested at 1:250. These columns summarize the data of Fig. 1 and 2.

d   The procedure followed was a modification of the plaque reduction test (Asano, Y., P. Albrecht, L.K. Vujcil, G. V. Quinnan, and M. Takahashi. 1983, "Evaluation of humoral immunity to varicella-zoster virus by an enhanced neutralization test and by the fluorescent antibody to membrane antigen test", Arch. Virol. 75:225-228). Serially diluted ascites fluids were mixed with an equal volume of cell-free VZV strain KMcC, either with or without 10% fresh guinea pig complement (C') and incubated for 1 hour at room temperature.  The test was set up such that 25 to 50 PFU was added per well, and four wells were assayed per antibody dilution.  The antibody-virus mixture (0.1 ml) was inoculated onto the

drained surface of Vero cells in 24-well (16-mm) plates. The virus was allowed to adsorb for 1 hour at 36°C, and the cells were refed and incubated for 7 days. The antibody titer was calculated as the reciprocal of the dilution which reduced the number of plaques by 50%.

[e] The FAMA test was performed essentially as previously described (Gershon, A. A., R. Raker, S. Steinberg, B. Topf-Ostein, and L. M. Drusin. 1976, "Antibody to varicella-zoster virus in parturient women and their offspring during the first year of life", Pediatrics 58:692-696; Williams, V., A. Gershon, and P. A. Brunell. 1974, "Serologic response to varicella-zoster membrane antigens measured by indirect immuno-fluorescene", J. Infect. Dis. 130:669-672), utilizing VZV-infected or uninfected FS-4 (human foreskin fibroblast) as indicator cells and fluorescein-labeled goat anti-mouse immuno-globulin (gamma and light chain specific; TAGO Inc.) as a second antibody. The antibody titer was calculated as the reciprocal of the dilution which gave detectable immunofluorescence.

[f] These clones demonstrated a preference in serological reactivity for gp92 over gp83.

[g] This antibody was nonreactive on Western blots.

## DESCRIPTION OF FIGURE 1

Figure 1. Electrophoretic analysis of immunoprecipitations of VZV polypeptides. Human diploid fibroblast (MRC-5) cells were grown at 36°C in Eagle basal metabolic medium with Earle salts and 10% fetal calf serum and were used as a virus host between passages 25 and 30 for the KMcC strain of VZV (Neff, B. J. et al., supra) which was passaged as a cell-associated stock. VZV-infected cells showing 50% cytopathic effect in 750-cm$^2$ roller bottles were labeled either with 10 ml of methionine-free Dulbecco modified Eagle medium plus 2% dialyzed fetal calf serum and 50 µCi of L-[$^{35}$S]methionine per ml (1,450 Ci/mmol; Amersham Corp.) or with 10 ml of Dulbecco modified Eagle medium containing 10% of the normal glucose concentration, 2% dialyzed fetal calf serum, and 10 µCi of D-[$^{14}$C]glucosamine hydro-chloride per ml (54 Ci/mmol Amersham Corp.]. After 24 hours, sonicated cell extracts were prepared by washing cell monolayers twice with 0.04M sodium phosphate-0.15M sodium chloride (phosphate-buffered saline), and 3 ml of SPG buffer (0.04 M sodium phosphate, 5% sucrose, 0.1% sodium glutamate) was added to each roller bottle. Cells were scraped into a centrifuge tube on ice, sonicated three times for 60 seconds each, and centrifuged at 2,000 x g for 10 minutes at 4°C. Sonicated cell extracts were stored at -70°C, unless used for virus purification, in which case they were processed immediately as follows. Extracts were layered onto 10 to 35% sucrose density gradients (in SPG buffer) and centrifuged for 25 minutes at 104,000 x g at 4°C. The lower virus band was removed from the side of the tube with a syringe, layered on a 35 to 55% sucrose density gradient (in SPG buffer) and centrifuged at 131,000 x g for 8 hours at 4°C. The lower band was collected, diluted with SPG buffer, and rebanded on a 35 to 55% sucrose density gradient.  The virus band then was collected, diluted in SPG buffer, pelleted at 131,000 x g for 2 hours at 4°C, resuspended in SPG buffer, and stored at -70°C until needed.  Electron microscopy of the purified material showed 80% enveloped virions. After purification of differentially labeled infected ($^3$H-labeled) and uninfected ($^{14}$C-labeled) cell lysates, the virus preparation was estimated to be purified 35-fold.  Immunoprecipitation analyses, sample preparations, and SDS-polyacrylamide gel electrophoresis were performed as described in the legend to Figure 3. Antigens used in these analyses were (A) [$^{35}$S]-methionine-labeled sonicated infected cell extracts, (B) [$^{14}$C]glucosamine-labeled sonicated infected cell extracts, or (C) [$^{35}$S]methionine-labeled purified virions.  These samples were immunoprecipitated by the following monoclonal antibodies (lanes): 1, control ascites fluid; 2, C8; 3, B1; 4; A1.  (D) Electrophoretic analysis of the major glycoprotein species of VZV; lanes: 1, lysate of purified [$^{14}$C]glucosamine-labeled virions; 2 and 3.  [$^{14}$C]-glucosamine-labeled sonicated cell extracts immuno-precipitated by (lane 2) convalescent zoster antiserum (VZV FAMA titer, 1:800) or (lane 3) normal human serum (VZV FAMA titer,  1:10).  Molecular weight markers are myosin (200.000 [200K]), phosphorylase b (92K), bovine serum albumin (68K), and ovalbumin (43K).

DESCRIPTION OF FIGURE 2

    Figure 2.  Western blot analysis of the reactivity of monoclonal antibodies to VZV polypeptides.  After sample preparation, as described in the legend to Figure 3, polypeptides were electrophoresed in 7.5% discontinuous SDS-polyacrylamide
gels under reducing conditions (Laemmli, U.K. 1970,
"Cleavage of structural proteins during the assembly
of the head of bacteriophage T4", Nature (London)
227: 680-685.) and transferred to nitrocellulose by
the method of Burnette (Burnette, W. N. 1981,
"Western blotting": Electrophoretic transfer of
proteins from sodium dodecyl sulfate-polyacrylamide
gels to unmodified nitrocellulose and radiographic
detection with antibody and radioiodinated protein
A", Anal. Biochem. 112:195-203.) All subsequent
incubations were performed at room temperature.
After transfer, the nitrocellulose was immersed in
buffer A (0.15 M NaCl, 50 mM Tris [pH 7.6], 0.1%
$NaN_3$) plus 20% agammaglobulin calf serum overnight, washed with incubation buffer (5% agammaglobulin calf serum in buffer A), and placed in
antisera of the desired dilution in incubation buffer
for 2 hours with rocking. The nitrocellulose then
was washed once with buffer A for 10 minutes, twice
with buffer A plus 0.05% Triton X-100 for 10 minutes
and once with buffer A again. When mouse antibodies
were being used, this step was followed by incubation
with a 1:1000 dilution of rabbit anti-mouse immunoglobulin G heavy and light chains in incubation
buffer for 1 hours, followed by the above washing
procedure. The blot then was incubated with 0.25 μCi
of $^{125}$I-protein A (New England Nuclear Corp.) per
ml in incubation buffer for 1 hour, washed as above,
dried, and autoradiographed. Purified virions (lane 1) or sonicated cell lysates (lane 2) were electrophoresed, blotted, and reacted with monoclonal antibody C5.

## DESCRIPTION OF FIGURE 3

Figure 3. Antigenic distinctiveness among VZV glycoproteins as demonstrated by cross-clearing immunoprecipitations. Formalin-fixed staph A cells (Bethesda Research Laboratories) were washed as described by Richert et al. (Richert, N. D., P. J. A. Davies, G. Jay and I. H. Pastan, 1979, "Characterization of an immune complex kinase in immunoprecipitates of avian sarcoma virus-transformed fibroblasts", J. Virol., 31: 695-706.), resuspended in BW buffer (0.1% SDS, 0.5% deoxycholate, 1% Triton-X 100, 0.01 M Tris [pH 8.0], 0.1 M NaCl, 0.002 M EDTA) at 6% (vol/vol) and stored at -70°C for immunoprecipitation of human antibodies. For immunoprecipitations with mouse antibodies, the staph A preparation was pelleted, rabbit anti-mouse immunoglobulin G heavy and light chains (Cappel Laboratories) were added, and the suspension was brought back to the original volume with phosphate-buffer saline. After the suspension was mixed for 2 hours at room temperature, it was washed twice with phosphate-buffered saline brought back to 6% (vol/vol) in 0.04 M Tris (pH 8.0)-0.02% $NaN_3$, and stored at 4°C for up to 1 month before use. Radiolabeled sonicated cell extracts or purified virions were brought to 1% Triton-X 100 and 0.5% deoxycholate and reclarified by centrifugation at 12,000 x g for 20 minutes at 4°C.

Antigen fractions containing ca. $10^6$ cpm were brought to a volume of 300 µl with 0.02 M Tris-hydrochloride (pH 7.4)-0.1 M NaCl-1% Triton-X 100-0.5% deoxycholate-0.005 M $MgCl_2$ and various volumes of ascites fluids (1 to 10 µl). The mixture was incubated for 2 hours at 4°C and 50 µl of the staph A preparation was added for a 1-hour incubation at 4°C with frequent mixing. The mixture then was pelleted by centrifugation at 18,000 x g for 3 minutes at 4°C, and the pellet was washed four times in 1 ml of BW buffer. After the last wash, the antigen was eluted by the addition of 40 µl of 2% SDS-4% mercaptoethanol-10% glycerol-0.063 M Tris phosphate (pH 7.5) and boiling for 3 minutes. Clearing immunoprecipitations were performed as described above, except that the antibody-treated supernatants from the first antigen-antibody-staph A reaction were saved and mixed with a second antibody for the second and third rounds of immunoprecipitation. Precipitated proteins were eluted in each cycle from the staph A as described above. Samples were electrophoresed in 7.5% discontinuous SDS-polyacrylamide gels under reducing conditions (Laemmli, U. K., supra). When immunoprecipitations were performed, the gels were fixed in 7% acetic-acid-25% methanol overnight, washed three times with 100 ml of dimethylsulfoxide for 15 minutes and impregnated with 2',5'-diphenyl-oxazole in dimethylsulfoxide (21 g/100 ml) for 1 hour. After the gels were washed in cold running water, they were dried in vacuo and fluorographed [$^{35}$S]methionine-labeled infected cell extracts were immunoprecipitated sequentially with monoclonal antibodies up to three times.  Each triplet of lanes (A through F) represents a single sequential experiment with the first (1), second (2), and third (3) monoclonal, respectively. Monoclonal antibodies used for each lane were: (A) 1, A1; 2, A1 and A1; 3, A1, A1 and A1; (B) 1, A1; 2, A1 and A1; 3, A1, A1 and B1; (C) 1, B1; 2, B1 and B1; 3, B1, B1 and A1; (D) 1, B1; 2, B1 and B1; 3, B1, B1 and B1; (E) 1, B1; 2, B1 and B1; 3, B1, B1 and C5; (F) 1, A1; 2, A1 and A1; 3, A1, A1 and C5.

### EXAMPLE 2
Purification of VZV gB Glycoprotein by Monoclonal
Antibody (B1) Immune Affinity Chromatography

Ascites fluids, carrying monoclonal antibody B1 described in Example 1, were harvested from mice. An equal volume of 0.15 M NaCl was added.  Then, a saturated $(NH_4)_2SO_4$ solution was added in an equal total volume and held at 4°C overnight.  This mixture was centrifuged at 4° for 10 minutes at 2000 rpm.  The pellet was resuspended in distilled $H_2O$ (2 mg/ml) and dialyzed overnight against coupling buffer (0.1M $NaHCO_3$, 0.5M NaCl, pH 8.4).  One gram of cyanogen bromide-activated Sepharose 4B (Pharmacia, Piscataway, N.J.) was swollen in 0.001N HCl then decanted into a 60 ml coarse sintered glass funnel. This was washed with 200 ml 0.001N HCl, then 50 ml coupling buffer.  The Sepharose was then mixed with 10 ml of monoclonal antibody solution and rotated for 2 hours at 23°C.  Then, 80 µl ethanolamine was added and the solution was rotated for 1 hour at 23°C.  The resin was poured into a disposable chromatography column (BioRad), drained and washed successively with 10 ml volumes of the following solutions:  1) coupling buffer; 2) 0.1M $Na_2HPO_4$; 0.5M NaCl, pH 8.2; 3) 0.1M NaOAc, 0.5 NaCl, pH 4.0; 4) 0.1M $NaHBO_4$, pH 8.2; 5) 3M KSCN; 6) 0.1M $NaHBO_4$, pH 8.2; then stored in 0.1M $NaHBO_4$, pH 8.2 at 4°C prior to use.

VZV glycoproteins were purified from MRC-5 human diploid fibroblasts which were infected with VZV to the extent of 80% cytopathic effect.  Cells in 750 $cm^2$ roller bottles were washed twice with 0.15M NaCl, 0.01M $Na_2HPO_4$, pH 7.2 and drained well. Ten ml of 50 mM Tris, pH 7.5, 2% Triton X-100, 4 mM phenylmethylsulfonylfluoride (PMSF) were incubated 15° to the bottle while rolling.  The same 10 ml then was used to successively extract 9 more roller bottles.  A fresh 10 ml aliquot of buffer was used to successively rinse the 10 roller bottles and pooled with the first aliquot, such that 20 ml of extract represent material from 10 roller bottles.  Extracts were stored at -70°C until use.

Extracts were thawed and dialyzed overnight at 4°C against 0.15M NaCl, 0.01M $Na_2HPO_4$, 0.05% Triton X-100, pH 7.2, then clarified by centrifuging at 1500 rpm for 15 minutes at 4°C.  20 ml of extract were added to 1 g of monoclonal antibody-coupled resin and incubated overnight at 4°C with shaking. The slurry was centrifuged for 15 minutes at 1500 rpm at 4°C and washed three times with 0.1M $NaHBO_4$, pH 8.2.  The glycoprotein was eluted by incubation at 23°C with 10 ml 3M KSCN.  The eluate was immediately dialyzed against 0.15M NaCl, 0.01M $Na_2HPO_4$, 0.05%

Triton X-100, pH 7.2 overnight at 4°C. The approximate yield of purified gB glycoprotein was 1 mg/10 roller bottles. The purity was shown as described in Example 10.

### EXAMPLE 3

Following substantially the same procedure as described in Example 2, except that monoclonal antibody A1 was used, prepared as described in Example 1, chromatographically pure gA VZV glycoprotein was obtained as shown as described in Example 10.

### EXAMPLE 4

Following substantially the same procedure as described in Example 2, except that monoclonal antibody C1 was used, prepared as described in Example 1, chromatographically pure gC VZV glycoprotein was obtained as shown as described in Example 10.

### EXAMPLES 5-7

The procedures for the purification of uninfected cell proteins which individually co-purify with VZV gA, gB or gC from VZV-infected cell lysates (by monoclonal antibodies A1, B1 or C1, respectively) were conducted substantially identical to Example 2, except for the change in antibody and the use of uninfected MRC-5 cells. Presence of these materials in the corresponding infected glycoprotein fractions is described in Example 10.

## EXAMPLE 8

**Purification of VZV Infected Cellular Glycoproteins
by Lectin Affinity Chromatography**

The lectin-coupled matrix (Ultragel Reactifs IBF lentil lectin, LKB) was preequilibrated in loading buffer (1M NaCl, 0.1% Triton X-100, 50 mM Tris, pH 7.5). Infected cell extract, prepared as described in Example 2 was loaded onto the column in a 200 ml volume (100 roller bottles). The column was washed extensively with loading buffer, and the glycoproteins were eluted with loading buffer containing 0.2M α-methylmannoside. The extract then was dialyzed overnight at 4°C against 0.15M NaCl, 0.01M $Na_2HPO_4$, 0.05% Triton X-100, pH 7.2. The approximate yield of purified glycoproteins was 50 mg/ 100 roller bottles. The characterization and identification of the product is given in Example 10.

## EXAMPLE 9

The example for purification of uninfected cell glycoproteins (by lectin-affinity chromatography) is absolutely identical to Example 8, except for the use of uninfected cells.

## EXAMPLE 10

**Immunological Activities of Purified VZV Glycoprotein**

Individual VZV glycoproteins and infected cell glycoproteins ("lectin") were purified as described in Examples 2, 3, 4 and 8. Each of the individual glycoprotein fractions was resolved by sodium dodecyl sulfate-polyacrylamide gel electrophoresis (SDS-PAGE) and silver-stained. These analyses demonstrated that each fraction was 75% pure. Several minor bands were observed in the affinity-purified uninfected glycoprotein fractions. These same minor bands were observed in the corresponding affinity-purified infected glycoprotein fraction, i.e., uninfected cell lysate bands from gA affinity chromatography were present in the purified infected gA glycoprotein fraction. Furthermore, molecular weights of the purified glycoproteins correlated perfectly with published data (Keller et al., ibid. see Example 1; Grose et al., ibid; Shiraki et al., ibid; Forghani et al., ibid; Okuno et al., ibid).

In another experiment, a lysate of $^{35}$S-methionine labelled VZV infected cells was used as the starting source for purification of each of the 3 glycoproteins and the lectin eluate. Purified glycoproteins were each immunoprecipitated with each of three monoclonal antibodies (A1, B1, C1) directed against each of the three glycoproteins gA, gB and gC respectively exactly as described (Keller et al., ibid, see Example 1). Immunoprecipitates were resolved by SDS-PAGE and autoradiographed. These analyses demonstrated that each purified glycoprotein preparation could be immunoprecipitated only by its homologous monoclonal antibody and that the lectin eluate contained all three glycoproteins in immunologically active form. Furthermore, there was a perfect correlation of the molecular weights of the immunoprecipitated proteins with the published data referred to above and contained in Example 1.

Each of the 3 purified glycoprotein preparations and the lectin eluate was adsorbed to alum and used to immunize guinea pigs in 10 mg doses. Sera from immunized animals was used to immunoprecipitate proteins from a lysate of $^{35}$S-methionine labelled VZV-infected cells exactly as described (Keller et al., ibid, see Example 1). In addition, sera were reacted with Western blots of SDS-PAGE resolved VZV-infected cell proteins as described in (Keller et al, see Example 1). By these analyses, it was found that each of the 3 purified glycoprotein immunogens was capable of eliciting antibodies reactive with the homologous glycoprotein by both Western blots and immunoprecipitation. In addition, reactivity was demonstrated of the antibodies to the homologous glycoproteins in the ELISA assay described in Examples 5 and 6. The lectin eluate elicited antibodies reactive with each of the 3 glycoproteins by immunoprecipitation, Western blot, and ELISA. In addition, each of the guinea pig sera were utilized in an in vitro neutralization assay as described (Keller et al., ibid). By this assay, each of purified gA, gB, and gC elicited neutralizing antibody. Therefore each of the 3 purified glycoprotein preparations and the lectin eluate was shown to be immunologically active by the criteria of 1) immunoprecipitation by monoclonal antibodies, 2) capability of eliciting virus neutralizing antibodies in vitro, and 3) capable of eliciting antibodies which demonstrate serological reactivity in Western blots and immunoprecipitations.

ASSAY OF ANTIBODIES IN HUMAN SERUM DIRECTED
AGAINST VZV GLYCOPROTEINS IN ELISA ASSAY

EXAMPLE 11

To each of 8 sets of four wells of a polystyrene multi-well assay plate wherein each well has a volume of about 0.3 ml there was added 0.15 ml of 0.54% $Na_2CO_3$, pH 9.8 and 0.1 µg of specific gA, gB or gC VZV glycoproteins or uninfected cell proteins. These protein preparations were obtained by the procedures described above utilizing immune-affinity chromatography (Examples 2-7) of VZV-infected or uninfected MRC-5 human diploid fibroblast cell lysates (3 preparations each) utilizing different monoclonal antibodies specifically reactive with each gA, gB or gC VZV glycoprotein family or by lectin-affinity chromatography (Examples 8, 9) of VZV-infected or uninfected cell lysates (1 preparation each) utilizing an immobilized lectin which was broadly cross-reactive with all VZV glycoproteins. The wells were covered and incubated at 4°C for 18 hours. The antigen solutions were decanted and the permanently glycoprotein coated wells were washed 3 times with 0.25 ml of PBS solution with 0.05% Tween 20[R]. The serum of the biological fluid to be tested was diluted 1:100 and 1:1000 in PBS containing 10% "globulin-poor" goat serum. 0.1 ml of each diluted serum was incubated with each coated well in a covered plate at 37°C for 1 hour. A negative control was obtained by adding to a third coated well 0.1 ml of the PBS buffer described above, washed as above. A positive control was obtained by adding to a fourth caprine coated well 0.1 ml of a 1:10,000 dilution of a VZV antibody-containing serum of known titer. The serum solutions are decanted and the empty wells were washed 3 times with 0.25 ml of PBS solution containing 0.05% Tween 20$^R$. 0.1 ml of goat anti-human IgG coupled to alkaline phosphatase (Tago, Inc., Burlingame, California) was incubated with each coated well in a covered plate at 37°C for 1 hour. The conjugate solutions were decanted and the empty wells were washed 3 times with 0.25 ml of PBS solution containing 0.05% Tween 20, and the buffer removed. To all five wells there was added 0.1 ml of substrate solution composed of 1 mg/ml of p-nitrophenyl phosphate/dissolved in 0.54% (wt./vol.) $Na_2CO_3$ buffer, pH 9.8, containing 0.02% (wt./vol.) $MgCl_2 \cdot 6H_2O$. This mixture was incubated at 22°C for 45 minutes. The enzyme-substrate reaction was stopped by the addition of 0.05 ml of 1N sodium hydroxide and the optical density (A) measured at 405 nm. The following results were obtained:

| Well | Serum Dilution | A(gA)* | A(gB)* | A(gC)* | A(lectin)* |
|------|----------------|--------|--------|--------|------------|
| 1 | 1:100 | 1.30 | 3.0+ | 2.70 | 2.39 |
| 2 | 1:1,000 | 0.19 | 0.16 | 0.42 | 0.62 |
| 3 | (negative control) | 0.0 | 0.0 | 0.0 | 0.0 |
| 4 | (positive control) | 1.50 | 0.60 | 2.50 | 2.60 |

* The optical density (A) for each of the 4 (gA, gB, gC, lectin) viral glycoprotein preparations was corrected by subtraction of the optical density for each of the matched uninfected cell glycoprotein controls.

3492P/1142A                    - 45 -                    17225Y

Since the "lectin" preparation contains amounts of gA, gB and gC which are approximately equimolar, the A(lectin) value is, to a very approximate extent, a rough weighted average of the A(gA), A(gB) and A(gC) values.

## ASSAY OF MURINE MONOCLONAL ANTIBODIES DIRECTED AGAINST VZV GLYCOPROTEINS IN ELISA ASSAY

### EXAMPLE 12

The procedure of Example 11 was repeated with two modifications. Firstly, murine monoclonal antibodies A1, B1 and C1 each specific for gA, gB, and gC VZV glycoproteins as prepared in Example 1 were used as the antibody source at 1:10,000 dilutions in "globulin-poor" goat serum. Secondly, the enzyme conjugate consisted of goat anti-mouse immunoglobulin IgG coupled to alkaline phosphatase (Tago, Inc., Burlingame, California). The following results were obtained with murine monoclonal antibody A1 (specific for VZV gA):

| Well | Ascites Fluid Dilution | A(gA) | A(gB) | A(gC) | A(lectin) |
|------|------------------------|-------|-------|-------|-----------|
| 1 | 1:10,000 | 0.84 | 0.0 | 0.0 | 0.03 |
| 2 | (negative control) | 0.0 | 0.0 | 0.0 | 0.0 |
| 3 | (positive control) | 1.50 | 0.60 | 2.50 | 2.60 |

The following results were obtained with murine monoclonal antibody B1 (specific for VZV gB):

| | Ascites | | | | |
|---|---|---|---|---|---|
| Well | Fluid Dilution | A(gA) | A(gB) | A(gC) | A(lectin) |
| 1 | 1:10,000 | 0.0 | 0.78 | 0.0 | 0.24 |
| 2 | (negative control) | 0.0 | 0.0 | 0.0 | 0.0 |
| 3 | (positive control) | 1.50 | 0.60 | 2.50 | 2.60 |

The following results were obtained with murine monoclonal antibody Cl (specific for VZV gC):

| | Ascites | | | | |
|---|---|---|---|---|---|
| Well | Fluid Dilution | A(gA) | A(gB) | A(gC) | A(lectin) |
| 1 | 1:10,000 | 0.0 | 0.0 | 0.84 | 0.08 |
| 2 | (negative control) | 0.0 | 0.0 | 0.0 | 0.0 |
| 3 | (positive control) | 1.50 | 0.60 | 2.50 | 2.60 |

These results indicate that the assay is specific for antibodies reactive with individual gA, gB or gC glycoproteins.

WHAT IS CLAIMED IS:

1. A VZV gA glycoprotein, also classified as gp105 or gp118 or gp1 glycoprotein, in its purified and non-denatured serologically active form.

2. A VZV gB glycoprotein, also classified as gp115-gp62-gp57 or gp2-gp5 or gp140-gp66, or 120K-118K-64,65K glycoprotein, or "disulphide-linked dimer", in its purified, non-denatured, serologically active form.

3. A VZV gC glycoprotein, also classified as gp92-gp83-gp52-gp45 or gp98-gp63 or gp3-gp4-gp6 or 90K-80K-60K glycoprotein, in its purified, non-denatured, serologically active form.

4. Uninfected cell protein in purified, non-denatured, serologically active form which co-purify with VZV gA, gB or gC, as also classified in Claims 1-3, from VZV-infected cell lysates by immune-affinity chromatography.

5. Purified infected cell VZV glycoproteins, purified by lectin-affinity chromatography, and containing gA and/or gB and/or gC glycoproteins, as also classified in Claims 1-3.

6. Purified uninfected cell glycoprotein, purified by lectin-affinity chromatography.

7.   A VZV diagnostic device comprising VZV gA glycoprotein, as also classified in Claim 1, in its purified, non-denatured, serologically active form permanently adhered to a solid surface.

8.   A diagnostic device comprising VZV gB glycoprotein, as also classified in Claim 2, in its purified, non-denatured, serologically active form, permanently adhered to a solid surface.

9.   A diagnostic device comprising VZV gC glycoprotein, as also classified in Claim 3, in its purified, non-denatured, serologically active form, permanently adhered to a solid surface.

10.   A method for determining the presence of antibodies to varicella-zoster virus (VZV) gA, gB and gC glycoproteins in a host mammalian biological fluid comprising the steps of:

(a)   permanently adhering at least one purified non-denatured, and serologically active gA, gB or gC VZV glycoprotein or mixture thereof, to a solid surface;

(b)   permanently adhering purified, non-denatured and serologically active uninfected cell proteins or glycoproteins or mixture thereof which co-purify with VZV gA, gB or gC glycoproteins, to a solid surface;

(c)   contacting the adhered glycoprotein on said solid surface from step (a) and adhered protein or glycoprotein on said solid surface from step (b) with the biological fluid, wherein antibodies to the glycoprotein, if present in the fluid, bind to said glycoprotein forming a glycoprotein-antibody complex;

(d) contacting the adhered glycoprotein on said solid surface from step (c) with an immunoglobulin-indicator complex, wherein said anti-immunoglobulin binds to said antibodies of said glycoprotein-antibody complex, if present from step (c), thereby forming a glycoprotein-antibody-anti-immunoglobulin-indicator complex, and wherein said indicator provides a measurable response of the binding event; and

(e) measuring the indicator response after conducting step (d) and correcting the response to the individual VZV glycoprotein or mixture thereof by subtraction of the response to its respective uninfected cell protein or glycoprotein control.

1/3

FIG. 1

—— 92 K

—— 68 K

—— 43 K

FIG. 2

FIG. 3

AI  A2  A3  BI  B2  B3  CI  C2  C3  DI  D2  D3  EI  E2  E3  FI  F2  F3

— 92 K

— 68 K

— 43 K

3/3

0211756

European Patent Office

**EUROPEAN SEARCH REPORT**

0211756

| DOCUMENTS CONSIDERED TO BE RELEVANT | | | EP 86401702.5 |

| Category | Citation of document with indication, where appropriate, of relevant passages | Relevant to claim | CLASSIFICATION OF THE APPLICATION (Int. Cl.4) |
|---|---|---|---|
| D,P X | JOURNAL OF VIROLOGY, vol. 57, no. 3, March 1986 (Washington DC) <br> A.J. DAVISON et al. "New Common Nomenclature of Glycoprotein Genes of Varicella-Zoster Virus and their Glycosylated Products" pages 1195-1196 <br> * Pages 1195,1196 * <br> -- | 1-3 | C 07 K 15/14 <br> G 01 N 33/569 <br> C 12 Q 1/70 <br> C 07 K 17/00 <br> A 61 K 39/35 |
| P,X | JOURNAL OF VIROLOGY, vol. 52, no. 1, October 1984 (Washington DC), <br> P.M. KELLER et al. "Three Major Glycoprotein Genes of Varicella-Zoster Virus whose products have Neutralization Epitopes" pages 293-297 <br> * Page 293; page 294, table 1 * <br> -- | 1-3 | |
| D,X | INFECTION AND IMMUNITY, vol. 40, no. 1, April 1983 (Washington DC) <br> CH. GROSE et al. "Monoclonal Antibodies Against three Major Glycoproteins of Varicella-Zoster Virus" pages 381-388 <br> * Page 381 * <br> -- | 1 | **TECHNICAL FIELDS SEARCHED (Int. Cl.4)** <br> C 07 K <br> G 01 N <br> C 12 Q <br> A 61 K |

The present search report has been drawn up for all claims

| Place of search | Date of completion of the search | Examiner |
|---|---|---|
| VIENNA | 07-11-1986 | WOLF |

## EUROPEAN SEARCH REPORT

| | DOCUMENTS CONSIDERED TO BE RELEVANT | | EP 86401702.5 |
|---|---|---|---|

**European Patent Office**

| Category | Citation of document with indication, where appropriate, of relevant passages | Relevant to claim | CLASSIFICATION OF THE APPLICATION (Int. Cl.4) |
|---|---|---|---|
| D,X | THE JOURNAL OF GENERAL VIROLOGY, vol. 61, 1982 (London) K. SHIRAKI et al. "Polypeptides of Varicella-Zoster Virus (VZU) and Immunological Relationship of VZV and Herpes Simplex Virus (HSV) pages 255-269 * Summary; page 261, table 2 * | 1,4 | |
| X | GB - A - 2 048 896 (PRIRODOVEDECKA FAKULTA UNIVERSITY KARLOVY) *Page 1, line 96 - page 2, line 7 * | 6 | |
| A | US - A - 4 457 865 (MILLER) * Claims 4,7 * | 6 | |
| | | | TECHNICAL FIELDS SEARCHED (Int. Cl.4) |

The present search report has been drawn up for all claims

| Place of search VIENNA | Date of completion of the search 07-11-1986 | Examiner WOLF |
|---|---|---|